(19) Europäisches Patentamt
European Patent Office
Office européen des brevets

(11) Veröffentlichungsnummer : **0 054 851**
**B1**

(12) # EUROPÄISCHE PATENTSCHRIFT

(45) Veröffentlichungstag der Patentschrift : **04.07.84**

(51) Int. Cl.³ : **C 07 C139/04**// C07C139/14

(21) Anmeldenummer : **81110309.2**

(22) Anmeldetag : **10.12.81**

(54) **Verfahren zum Abtrennen von Schwefelsäure aus dem bei der Sulfoxidation von Paraffinen anfallenden Reaktionsgemisch.**

(30) Priorität : **19.12.80 DE 3048058**

(43) Veröffentlichungstag der Anmeldung : **30.06.82 Patentblatt 82/26**

(45) Bekanntmachung des Hinweises auf die Patenterteilung : **04.07.84 Patentblatt 84/27**

(84) Benannte Vertragsstaaten : **AT BE CH DE FR GB IT LI NL SE**

(56) Entgegenhaltungen :
**FR-A- 2 367 740**
**FR-A- 2 376 842**
**US-A- 3 720 707**

(73) Patentinhaber : **HOECHST AKTIENGESELLSCHAFT**
**Postfach 80 03 20**
**D-6230 Frankfurt am Main 80 (DE)**

(72) Erfinder : **Mees, Bernhard, Dr.**
**Gräfliche Strasse 31**
**D-6239 Eppstein/Taunus (DE)**
Erfinder : **Ramloch, Herbert, Dr.**
**Eichhornweg 3**
**D-6232 Bad Soden am Taunus (DE)**

**EP 0 054 851 B1**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Jouve, 18, rue St-Denis, 75001 Paris, France

**Beschreibung**

Bei der Sulfoxidation von linearen Paraffinen nach dem Licht-Wasser-Verfahren, d. h. mit $SO_2$ und $O_2$ in Gegenwart von Wasser und mit UV-Licht, entsteht nach der Abtrennung von ungelöstem Paraffin eine Mischung aus Paraffin, Wasser, Alkansulfonsäure und Schwefelsäure. Die Zusammensetzung dieser Mischung ist abhängig von der Kettenlänge des eingesetzten Paraffins. Bei der Sulfoxidation eines Paraffins der Kettenlänge $C_{12}$-$C_{18}$ und einem Maximum bei $C_{14}$-$C_{17}$, erhält man beispielsweise nach dem Austreiben von $SO_2$ eine Mischung etwa folgender Zusammensetzung : 37,5 % Wasser, 32 % Paraffin, 23 % Alkansulfonsäure und 7,5 % Schwefelsäure.

Diese Mischung ist eine völlig klare Lösung und soll in den folgenden Ausführungen als Extrakt bezeichnet werden. Es handelt sich dabei wahrscheinlich um eine Mikroemulsion von Paraffin in der wässrigen Schwefelsäure, wobei die Alkansulfonsäure als Solubilisierungsmittel wirkt.

Für die Gewinnung von reinem Alkansulfonsäure-Na-Salz, das als leicht biologisch abbaubares Tensid große Bedeutung besitzt, ist die Anwesenheit von Schwefelsäure sehr störend, da bei der Neutralisation ein entsprechender Anteil Natriumsulfat entsteht.

Es ist deshalb in der Vergangenheit immer wieder der Versuch unternommen worden, den Extrakt so zu beeinflussen, daß eine Abtrennung der Schwefelsäure erfolgt. So ist z. B. bekannt, daß eine Erwärmung des Extraktes auf ca. 90 °C zu einer teilweisen Abtrennung der Schwefelsäure führt. Die technisch erreichbare Abtrennung liegt jedoch nur bei ca. 35 % der Gesamtmenge $H_2SO_4$. Es ist ebenso bekannt, dem Extrakt schwach polare Alkohole mit mindestens 5 Kohlenstoffatomen zuzusetzen, um eine Entmischung in eine organische Phase, welche die Gesamtheit der Sulfonsäure enthält und in eine wässrige Phase, die fast die gesamte Schwefelsäure enthält, herbeizuführen. Der Nachteil bei diesem Verfahren liegt einerseits in der großen Menge an Alkohol, die dem Extrakt zugesetzt werden muß (zwischen 20-80 g Alkohol pro 100 g Extrakt) und andererseits in der schwierigen Trennung des Alkohols vom Paraffin. Es ist schließlich bekannt, dem Extrakt schwach polare organische Lösungsmittel zuzusetzen wie Äther, Ketone, Ester und aliphatische Ketoester. Der Nachteil auch bei diesem Verfahren liegt in der großen Menge an Lösungsmitteln zur Abtrennung der Schwefelsäure. Diese Einsatzmenge wird in der Literatur mit 20-100 g pro 100 g Extrakt angegeben.

Es stellte sich daher die Aufgabe, eine möglichst quantitative Abtrennung der Schwefelsäure zu erreichen unter Zugabe möglichst geringer Mengen eines Hilfsmittels. Es wurde nun gefunden, daß man dieses Ziel erreichen kann, indem man dem bei der Sulfoxidation von Paraffinen anfallenden Reaktionsgemisch ein organisches Amin zugibt und die sich dabei abscheidende Phase aus Wasser und Schwefelsäure abtrennt.

Man nimmt für die Abtrennung der Schwefelsäure organische Amine der Formel

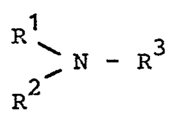

wobei $R^1$ $C_3$-$C_{18}$-Alkyl, $C_5$-$C_8$-Cycloalkyl, Phenyl, das durch 1 bis 3 $C_1$-$C_4$-Alkylgruppen oder eine $C_1$-$C_9$-Alkylgruppe substituiert sein kann, oder wenn $R^3$ eine Gruppe der Formel —X—$NR^1R^2$ darstellt, auch Wasserstoff, $R^2$ die gleiche Bedeutung hat wie $R^1$ und zusätzlich Wasserstoff, $R^3$ die gleiche Bedeutung hat wie $R^1$ und zusätzlich Wasserstoff oder eine Gruppe der Formel —X—$NR^1R^2$ und X Phenylen oder $C_8$-$C_{12}$-Alkylen bedeutet. Besonders bevorzugt sind solche Amine der obigen Formel, die nur Alkyl- bzw. Alkylengruppen, insbesondere geradkettige Alkyl- bzw. Alkylengruppen enthalten.

Geeignete Amine dieser Art sind aliphatische Mono-, Di- und Trialkylamine, wobei die Alkylreste sowohl geradkettig wie auch verzweigt sein können. Ebenso geeignet sind entsprechende cycloaliphatische Mono-, Di- und Trialkylamine. Auch aromatische Amine wie z. B. Xylidin oder Äthylanilin zeigen die gewünschte Wirkung ebenso wie auch längerkettige aliphatische Diamine.

Amine, die eine besonders gute Wirksamkeit zeigen, sind Hexylamin, Heptylamin, Octylamin, Diisobutylamin, Dibutylamin, Dipentylamin, Dihexylamin, Tributylamin, Dibutylmethylamin, Tripentylamin, Trihexylamin, Cyclohexylamin, Dicyclohexylamin, Tricyclohexylamin, Diamino-octan-1,8-Anilin, o-Toluidin, p-Toluidin, Xylidin, 2-Äthylanilin, o-Diaminobenzol, Aminodiphenyl und p-Nonylanilin.

Diese Amine werden in der Regel in einer Menge von 1 bis 5, vorzugsweise 2 bis 4 Gewichtsteilen bezogen auf 100 Gewichtsteile Extrakt dem rohen Sulfonierungsgemisch (Extrakt) zugegeben. Zuvor wird der Extrakt noch von darin gelösten Restmengen an $SO_2$ befreit. Die Abscheidung der Unterphase aus Wasser und Schwefelsäure erfolgt unter Zugabe des Amins schon bei Raumtemperatur. Um eine möglichst vollständige Abtrennung der Schwefelsäure zu erreichen, ist es vorteilhaft, den Extrakt auf ca. 20 bis 130, insbesondere 90 bis 95 °C aufzuheizen. Nach der Zugabe des Amins läßt man das Gemisch noch ca. 30 bis 60 Minuten stehen und trennt dann die entstandene Unterphase ab, die aus ca. 20 %iger wässriger Schwefelsäure besteht und praktisch die gesamte, ursprünglich im Extrakt vorhandene Menge an Schwefelsäure enthält. Diese Schwefelsäure kann in einem gesonderten Verfahren weiter aufgearbeitet werden. Die zurückbleibende obere Phase besteht aus dem nicht umgesetzten Rest-Paraffin und der

Alkansulfonsäure, wobei ein Teil dieser Alkansulfonsäure in Form ihres Salzes mit dem als Phasentrennmittel zugegebenen Amin vorliegt.

Das oben beschriebene Verfahren zeichnet sich besonders dadurch aus, daß man eine vollständige Abtrennung der Schwefelsäure erreicht, wobei aber die hierzu benötigte Menge an Amin etwa um den Faktor 10 niedriger ist als bei den bisher zu diesem Zweck benutzten schwach polaren Lösungsmitteln. Ein weiterer Vorteil liegt darin, daß die organischen Amine nicht mit der Schwefelsäure in die wässrigen Unterphase wandern, sondern vollständig mit der Alkansulfonsäure in der Oberphase verbleiben. Das erfindungsgemäße Verfahren wird insbesondere zur Trennung von rohen Sulfonierungsgemischen angewandt, die aus der Sulfoxidation von n-Paraffinen mit 7 bis 30, vorzugsweise 10 bis 20 C-Atomen stammen.

Beispiel 1

Zu 100 Gewichtsteilen von einem entgasten Extrakt mit folgender Zusammensetzung :

| | |
|---|---|
| Wasser : | 37,5 % |
| Paraffin : | 32 % |
| Alkansulfonsäure : | 23 % |
| Schwefelsäure : | 7,5 % |

gibt man unter Rühren 3 g Gew.-Teile Di-n-pentylamin bei 95 °C und rührt 5 Minuten nach. Anschließend hält man die Mischung etwa 45 Minuten in einem beheizten Scheidetrichter, wobei sich der Extrakt in zwei Phasen trennt. Danach läßt man die farblose wässrige Schwefelsäure als Unterphase ab. Man erhält 35 Gewichtsteile einer ca. 20 %igen Schwefelsäure, deren Gehalt titrimetrisch bestimmt wird. Bezogen auf die im Extrakt vorhandene Schwefelsäure werden 92 % abgetrennt.

Beispiel 2

Zu 100 Gewichtsteilen des entgasten Extrakts nach Beispiel 1 gibt man bei Raumtemperatur 3,5 g Tri-n-butylamin und läßt 10 Minuten rühren. Anschließend gibt man die Mischung in einen heizbaren Scheidetrichter und trennt entsprechend dem Beispiel 1 die Schwefelsäure als wässrige Unterphase ab. Man erhält 35,5 g wässrige Schwefelsäure, die 94,6 % der ursprünglich im Extrakt vorhandenen Menge an Schwefelsäure enthält.

Beispiel 3

100 Gewichtsteile des entgasten Extraktes nach Beispiel 1 werden mit 3,5 Gewichtsteilen Dicyclohexylamin in Form von 9,5 Gewichtsteilen Alkansulfonsäure-Dicyclohexylammoniumsalz bei 90 °C verrührt und dann entsprechend dem Beispiel 1 aufgearbeitet. Man erhält nach der Abtrennung 35,2 g wässrige Schwefelsäure, die 93 % der ursprünglich im Extrakt vorhandenen Menge an Schwefelsäure enthält.

Beispiel 4

100 Gewichtsteile des entgasten Extraktes nach Beispiel 1 werden durch Erhitzen auf 90 °C vorgetrennt, wobei 33 % der vorhandenen Schwefelsäure in Form einer ca. 23 %igen Schwefelsäure in der Unterphase verbleiben welche abgetrennt wird. Anschließend gibt man 4 Gewichtsteile n-Hexylamin zu und arbeitet gemäß Beispiel 1 weiter auf. Man erhält als Unterphase weitere 60 % der Schwefelsäure, so daß 93 % der insgesamt vorhandenen Menge an Schwefelsäure abgetrennt werden.

Weitere Beispiele, die analog zu den Beispielen 1-4 gemacht wurden sind im folgenden in Form einer Tabelle zusammengefaßt. Die in allen Beispielen angegebenen Mengen an Amin sind so gewählt, daß der Gehalt an Restschwefelsäure der jetzigen Spezifikation für handelsübliche Alkansulfonate entspricht. Durch Zugabe einer größeren Menge an Amin läßt sich dieser Restgehalt in allen Fällen weiter verringern.

| Eingesetztes Amin | Gewichtsteile Amin pro 100 Gew.T.Extrakt | Gew.-% abgetrennte Schwefelsäure |
|---|---|---|
| n-Pentylamin | 3,5 | 92 |
| n-Heptylamin | 3,5 | 91 |
| Cyclohexylamin | 4 | 93 |
| n-Octylamin | 4 | 92 |
| n-Laurylamin | 4 | 90 |

(Siehe Fortsetzung Seite 4 f.)

(Fortsetzung)

| Eingesetztes Amin | Gewichtsteile Amin pro 100 Gew.T.Extrakt | Gew.-% abgetrennte Schwefelsäure |
|---|---|---|
| n-Stearylamin | 4 | 90 |
| i-Hexylamin | 4 | 92 |
| i-Heptylamin | 3,5 | 89 |
| i-Octylamin | 3,5 | 89 |
| i-Tridecylamin | 3,5 | 90 |
| Dibutylamin | 3,0 | 93 |
| Diisobutylamin | 3,0 | 92 |
| Di-n-hexylamin | 3,5 | 91 |
| Distearylamin | 4 | 89 |
| Dibutylmethylamin | 3,0 | 92 |
| Diaminooctan 1.8 | 3,5 | 88 |
| Diaminoundecan 1.11 | 4,0 | 92 |
| Anilin | 4,0 | 88 |
| o-Toluidin | 3,5 | 89 |
| p-Toluidin | 3,5 | 89 |
| Xylidin 1.3 | 3,5 | 91 |
| p-Nonylanilin | 3,5 | 92 |
| 2-Athylanilin | 3,0 | 93 |
| o-Diaminobenzol | 3,5 | 89 |
| Aminodiphenyl | 3,5 | 90 |

**Ansprüche**

1. Verfahren zur Abtrennung von Schwefelsäuren aus dem bei der Sulfoxidation von Paraffinen anfallenden Reaktionsgemisch, dadurch gekennzeichnet, daß man das Reaktionsgemisch versetzt mit einem organischen Amin der Formel

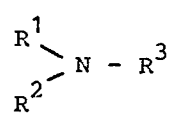

wobei $R^1$ $C_3$-$C_{18}$-Alkyl, $C_5$-$C_8$-Cycloalkyl, Phenyl, das durch 1 bis 3 $C_1$-$C_4$-Alkylgruppen oder eine $C_1$-$C_9$-Alkylgruppe substituiert sein kann, oder wenn $R^3$ eine Gruppe der Formel —X—$NR^1R^2$ darstellt, auch Wasserstoff, $R^2$ die gleiche Bedeutung hat wie $R^1$ und zusätzlich Wasserstoff, $R^3$ die gleiche Bedeutung hat wie $R^1$ und zusätzlich Wasserstoff oder eine Gruppe der Formel —X—$NR^1R^2$ und X Phenylen oder $C_8$-$C_{12}$-Alkylen bedeutet, und die sich dabei abscheidene Phase aus Wasser und Schwefelsäure abtrennt.

2. Verfahren nach Anspruch 1, dadurch gekennzeichnet, daß man das Reaktionsgemisch versetzt mit einem organischen Amin der Formel

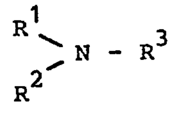

wobei $R^1$ $C_3$-$C_{18}$-Alkyl oder, wenn $R^3$ eine Gruppe der Formel —X—$NR^1R^2$ darstellt, auch Wasserstoff, $R^2$ die gleiche Bedeutung hat wie $R^1$ und zusätzlich Wasserstoff, $R^3$ die gleiche Bedeutung hat wie $R^1$ und zusätzlich Wasserstoff oder eine Gruppe der Formel —X—$NR^1R^2$ und X $C_8$-$C_{12}$-Alkylen bedeutet.

3. Verfahren nach Anspruch 1, dadurch gekennzeichnet, daß man das Reaktionsgemisch versetzt mit einem organischen Amin der Formel

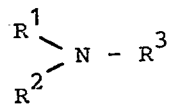

wobei $R^1$ geradkettiges $C_3$-$C_{18}$-Alkyl oder wenn $R^3$ eine Gruppe der Formel —$XNR^1R^2$ darstellt, auch Wasserstoff, $R^2$ die gleiche Bedeutung hat wie $R^1$ und zusätzlich Wasserstoff, $R^3$ die gleiche Bedeutung hat wie $R^1$ und zusätzlich Wasserstoff oder eine Gruppe der Formel —X—$NR^1R^2$ und X geradkettiges $C_8$-$C_{12}$-Alkylen bedeutet.

4. Verfahren nach Anspruch 1, dadurch gekennzeichnet, daß die Amine in einer Menge von 1-5 Gewichtsteilen, vorzugsweise 2-4 Gewichtsteilen je 100 Gewichtsteile Extrakt eingesetzt werden.

5. Verfahren nach Anspruch 1, dadurch gekennzeichnet, daß die Abtrennung der Schwefelsäure nach Zugabe der Amine bei einer Temperatur von 20-130 °C, vorzugsweise bei einer Temperatur von 90-100 °C vorgenommen wird.

**Claims**

1. A process for the removal of sulfuric acid from the reaction mixture formed in the sulfoxidation of paraffins, which process is characterized by adding an organic amine of the formula

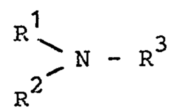

in which $R^1$ denotes $C_3$-$C_{18}$-alkyl, $C_5$-$C_8$-cycloalkyl or phenyl which can be substituted by 1 to 3 $C_1$-$C_4$-alkyl groups or by a $C_1$-$C_9$-alkyl group, or, if $R^3$ represents a group of the formula —X—$NR^1R^2$, also denotes hydrogen, $R^2$ has the same meaning as $R^1$ and additionally denotes hydrogen, $R^3$ has the same meaning as $R^1$ and additionally denotes hydrogen or a group of the formula —X—$NR^1R^2$ and X denotes phenylene or $C_8$-$C_{12}$-alkylene, to the reaction mixture and removing the phase, composed of water and sulfuric acid, which then separates out.

2. A process as claimed in Claim 1, wherein an organic amine of the formula

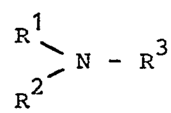

in which $R^1$ denotes $C_3$-$C_{18}$-alkyl or, if $R^3$ represents a group of the formula —X—$NR^1R^2$, also denotes hydrogen, $R^2$ has the same meaning as $R^1$ and additionally denotes hydrogen, $R^3$ has the same meaning as $R^1$ and additionally denotes hydrogen or a group of the formula —X—$NR^1R^2$ and X denotes $C_8$-$C_{12}$-alkylene, is added to the reaction mixture.

3. A process as claimed in Claim 1, wherein an organic amine of the formula

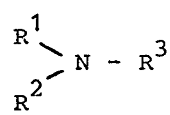

in which $R^1$ denotes straight-chain $C_3$-$C_{18}$-alkyl or, if $R^3$ represents a group of the formula —$XNR^1R^2$, also denotes hydrogen, $R^2$ has the same meaning as $R^1$ and additionally denotes hydrogen, $R^3$ has the same meaning as $R^1$ and additionally denotes hydrogen or a group of the formula —X—$NR^1R^2$ and X denotes straight-chain $C_8$-$C_{12}$-alkylene, is added to the reaction mixture.

4. A process as claimed in Claim 1, wherein the amines are employed in a quantity of 1-5 parts by weight, preferrably 2-4 parts by weight, per 100 parts by weight of extract.

5. A process as claimed in Claim 1, wherein the removal of the sulfuric acid, after the addition of the amines, is effected at a temperature of 20-130 °C, preferably at a temperature of 90-100 °C.

**Revendications**

1. Procédé pour séparer l'acide sulfurique du mélange réactionnel obtenu lors de la sulfoxydation de paraffines, procédé caractérisé en ce qu'on ajoute au mélange réactionnel une amine organique répondant à la formule

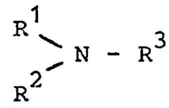

dans laquelle $R^1$ désigne un radical alkyle en $C_3$ à $C_{18}$, un radical cycloalkyle en $C_5$ à $C_8$, un radical phényle éventuellement porteur de 1 à 3 groupes alkyles en $C_1$ à $C_4$ ou d'un groupe alkyle en $C_1$ à $C_9$, ou, lorsque $R^3$ représente un radical de formule —X—$NR^1R^2$, peut aussi désigner l'hydrogène, $R^2$ a la même signification que $R^1$ et désigne en outre l'hydrogène, $R^3$ a la même signification que $R^1$ et désigne en outre l'hydrogène ou un radical de formule —X—$NR^1R^2$ et X désigne un radical phénylène ou alkylène en $C_8$ à $C_{12}$, et en ce qu'on sépare la phase d'eau et d'acide sulfurique qui se dépose.

2. Procédé suivant la revendication 1, caractérisé en ce qu'on ajoute au mélange réactionnel une amine organique répondant à la formule

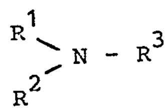

dans laquelle $R^1$ désigne un radical alkyle en $C_3$ à $C_{18}$, ou, lorsque $R^3$ représente un groupe de formule —X—$NR^1R^2$, désigne également l'hydrogène, $R^2$ a la même signification que $R^1$ et désigne en outre l'hydrogène, $R^3$ a la même signification que $R^1$ et désigne en outre l'hydrogène ou un groupe de formule —X—$NR^1R^2$ et X représente un alkylène en $C_8$ à $C_{12}$.

3. Procédé suivant la revendication 1, caractérisé en ce qu'on ajoute au mélange réactionnel une amine organique répondant à la formule

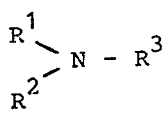

dans laquelle $R^1$ désigne un groupe alkyle linéaire en $C_3$ à $C_{18}$ ou, lorsque $R^3$ représente un groupe de formule —X—$NR^1R^2$, désigne également l'hydrogène, $R^2$ a la même signification que $R^1$ et désigne en outre l'hydrogène, $R^3$ a la même signification que $R^1$ et désigne en outre l'hydrogène ou un groupe de formule —X—$NR^1R^2$ et X désigne un alkylène linéaire en $C_8$ à $C_{12}$.

4. Procédé suivant la revendication 1, caractérisé en ce que les amines sont utilisées en une quantité de 1 à 5 parties en poids, de préférence de 2 à 4 parties en poids, pour 100 parties en poids d'extrait.

5. Procédé suivant la revendication 1, caractérisé en ce que la séparation de l'acide sulfurique est effectuée, après addition des amines, à une température de 20 à 130 °C, de préférence de 90 à 100 °C.